# EUROPEAN PATENT APPLICATION

(11) **EP 3 552 569 A1**
(43) Date of publication of application: **16.10.2019**
(21) Application number: 19175744.2
(22) Date of filing: 23.03.2016
(51) Int. Cl.: A61B 18/14

(54) **VESSEL ABLATION SYSTEM WITH ADJUSTABLE ABLATION TERMINAL**

(30) Priority: 27.03.2015 GB 201505284
(62) Divisional of application: 16275048.3
(71) Applicant: Cook Medical Technologies LLC, Bloomington, IN 47404 (US)
(72) Inventor: Pedersen, Per Elgaard, 4690 Haslev (DK); Torp, Allan, 4632 Bjaeverskov (DK); Butzbacker, Raimo Urban, 4690 Haslev (DK); Paamand, Rune T., 3050 Humlebaek (DK)
(74) Representative: Williams Powell

(57) **Abstract**

An RF ablation system (10) includes a catheter (12) having a lumen therein and a first electrode (18). A second electrode (20) is disposed around the catheter (12) proximate the distal end (14) of the catheter (12) and spaced from the first electrode (18) by an insulating spacer which may be a part (36) of the catheter (12) or a sleeve (42) on the first electrode (18). The first electrode (18) is able to slide in and out of the catheter (12) in order to vary the exposed length of the first electrode (18) and as a result to alter the energy density at the first electrode (18) in order to exhibit different heating effects. A control unit (24) controls current through the electrodes (18, 20) and also the exposed length of the first electrode (18). The second electrode (20) has a surface area at least twice that of the first electrode (18) in order to act as a dispersive of electrode. As a consequence, negligible heat is generated at the second electrode (20) and therefore no blood clotting or other ablation material is formed over the catheter (12).

## Description

### Technical Field

The present invention relates to ablation apparatus for effecting ablation within a vessel of a patient, for causing blood clotting or for treating an organ or medical condition.

### Background Art

Ablation systems are known in the art, for use for example in closing off a vessel. These may work by heating the blood in the vessel to a temperature which causes blood clotting or by ablating the tissue of the vessel wall, which causes collapse of the vessel and fusing of the wall tissue to itself thereby to seal the vessel in a closed condition.

There are a number of systems known in the art for effecting ablation in or of a vessel. A popular method makes use of RF electrical energy to heat the blood or vessel wall by means of a bipolar or a monopolar system. A bipolar system provides feed and return electrodes at the distal end of an elongate carrier which is inserted endoluminally into the patient from a percutaneous entry point. RF electrical energy passes to and from the terminals at the distal end of the carrier, heating the surrounding blood or vessel tissue as a result of electrical resistance of the blood plasma or vessel tissue. A monopolar device has a carrier similar to that of a bipolar system but which has a single electrical terminal at the distal end of the elongate carrier and is also provided with an external terminal, often in the form of a conductive pad, which in use is disposed against the patient's body at a location close to where the distal end of the carrier is positioned. A bipolar system makes use of an electrical circuit between the two electrodes through the patient's blood, whereas a monopolar system creates an electrical circuit from within the vessel through the patient's body to the outlying electrical pad.

A problem which has been encountered with ablation systems lies with managing the heat generated during the process and resultant undesired damage to adjacent tissues or the patient's organs, particularly those at or proximate the internally positioned electrode.

Examples of ablation systems are disclosed in US-5,403,311 , US-5,507,725, US-6,068,626, US-2002/0,022,834, US-2013/0,030,385 and US-2013/0,035,686.

### Summary of the Invention

The present invention seeks to provide improved ablation apparatus and an improved method of effecting ablation in a patient.

According to an aspect of the present invention, there is provided medical ablation apparatus including: an endoluminal delivery catheter having a lumen therein, an outer surface and a distal end wall, at least the outer surface of the catheter being made of an electrically non-conductive material; at least one elongate electrically conductive first terminal disposed in the lumen of the catheter, the first terminal having a first surface area; an electrically conductive second terminal disposed around the outer surface of the catheter and spaced from the first terminal so as to provide an insulating gap between the first and second terminals, the second terminal having a surface area a multiple of the surface area of the first terminal. In an embodiment, the second terminal is spaced from the distal end wall of the catheter.

The arrangement and sizing of the electrical terminals causes heat concentration at the first terminal, or electrode, and as a result for ablation to occur at the first terminal.

Preferably, the second terminal is an electrically dispersive electrode. In this regard, the second terminal may have a surface area at least twice the surface area of the first electrode, more preferably at least three times the surface area of the first electrode. It has been discovered that by using a second electrode with a surface area around three times, or more, than that of the first electrode, virtually no heat is generated at the second electrode, leading to the ability to control the location of heat generation as well as avoiding ablated material coating the catheter. This latter features facilitates the removal of the device from within the patient without disturbing the ablated material.

In a preferred embodiment, the electrically conductive first terminal is slidably disposed in the lumen of the catheter. This enables the operative length of the first electrode to be varied, which can alter the heating characteristics of the apparatus, as described in detail in the specific description which follows. This can enable the apparatus to treat different conditions, for example to cause blood clotting, vessel sealing, destruction of damaged or diseased tissue such as tumours, and so on.

For this purpose, the apparatus may include a control system coupled to the first and second electrically conductive terminals, the control system including a motor connected to the first terminal and operable to move the first terminal relative to the second terminal, thereby to change an exposed length of the first terminal.

The control system may include a control input for inputting a vessel size, the control unit being configured to control the motor to adjust the exposed length of first terminal in dependence upon vessel size. The control system can be configured to enable a user, typically a clinician, to adjust the apparatus on the basis of measured vessel size, for example. An adjustment could also be made on the basis of flow speed or of a combination of vessel size and flow speed.

In a practical embodiment, the control system includes a look-up table of vessel size and associated desired exposed length of the first electrode. The look-up table may also or in the alternative have indications of blood flow volume or speed, or a combination of blood flow and vessel size.

In some embodiments, at maximum exposure of the first terminal, the second terminal has a surface area a multiple of the surface area of the first terminal, for example at least twice or at least three times the surface area of the first terminal, for example is an electrically dispersive electrode.

In a preferred embodiment, the first electrode is an electrically conductive wire. This can provide a very small diameter device useful for placement in small diameter vessels, for example, for neurological or cerebral treatments.

The first electrode may have an exposed length from 5 to 20 millimetres, preferably from 8 to 15 millimetres. It will be appreciated that the first electrode may be adjustable to a length within these ranges. In some embodiments the first electrode may be entirely retractable into the catheter.

In some embodiments, the second terminal has a surface area a multiple of, for example at least twice or at least three times, the surface area of the first terminal, for example is an electrically dispersive electrode, during operation of the apparatus, such as during ablation.

In an embodiment, the second electrode is an electrically conductive ring disposed around the catheter. It may be an electrically conductive ring of braided conductive wires, an electrically conductive winding of conductive strip material disposed helically around the catheter, a sleeve or other structure.

The first and second terminals are advantageously spaced from one another by an insulating gap of at least 2 millimetres, preferably of at least 3 millimetres. In practice, this may be provided by spacing the second terminal from the distal end wall of the catheter, while in other embodiments there may be an insulator between the first and second terminals, such as a sleeve over a part of the first terminal.

According to an aspect of the invention, there is provided a method of effecting ablation using an apparatus as above, including:
configuring the first terminal to expose a desired operative length; and
operating the first and second terminals to effect ablation.

The method can include operating a motor unit such as above to move the first terminal to expose a desired operative length, for example in dependence on vessel size.

The method can incorporate other features described in connection with the apparatus.

Other features and advantages are described below in connection with the preferred embodiments.

### Brief Description of the Drawings

Embodiments of the present invention are described below, by way of example only, with reference to the accompanying drawings, in which:
Figure 1 is a schematic diagram of an embodiment of medical ablation system;
Figure 2 is an enlarged view of the distal end of the electrical terminal assembly for the system of Figure 1;
Figure 3 is an enlarged view of the distal end of another embodiment of electrical terminal assembly for the system of Figure 1, in which the first electrical terminal has an insulation sleeve over a part of its length;
Figures 4A to 4C show different examples of the electrical terminal disposed around a catheter of the electrical terminal assembly;
Figures 5A and 5B show the heating distribution of the system with different lengths of the first electrical terminal; and
Figure 6 is a schematic diagram of an embodiment of control unit for the system of Figure 1.

### Description of the Preferred Embodiments

There is described below and shown in the accompanying schematic diagrams a variety of embodiments of RF ablation apparatus for use in occluding a body vessel. The preferred embodiment achieves this by causing coagulation of blood by heat generation, in order to close off the vessel. The apparatus could equally be used to effect ablation by means of heating the vessel wall, which can cause shrinkage of the vessel and fusing of the vessel wall tissue into a closed condition. Other embodiments can generate ablation heat deep into tissue, in order for instance to destroy a tumor or other diseased tissue or organ material.

The apparatus in its preferred embodiments can provide a very small diameter ablation element suitable for very small diameter vessels. The skilled person will appreciate that the teachings herein can be scaled up for use in larger sized vessels.

The drawings are schematic only and the various elements are not necessarily shown to scale or with all of the components typically used in such apparatus. Any secondary elements and features have been omitted for the sake of clarity.

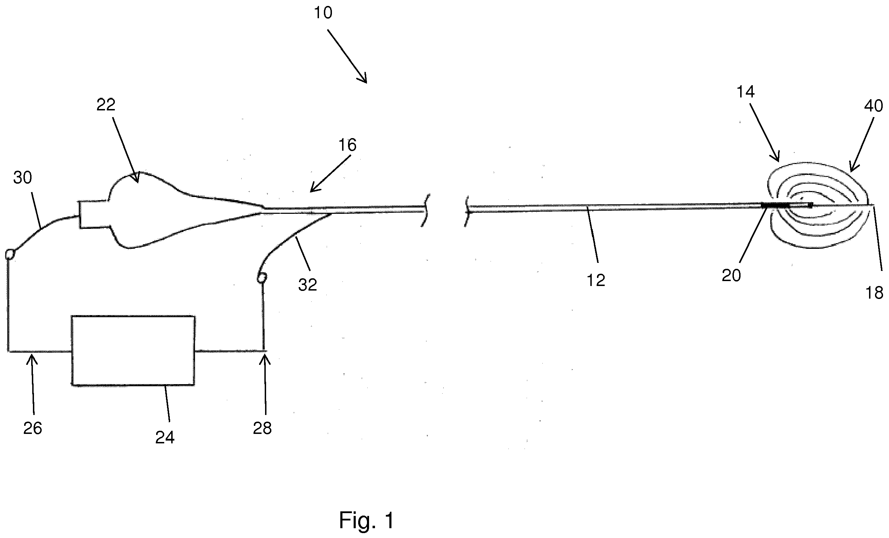

Referring first to Figure 1, this shows in schematic form the principal components of an RF ablation apparatus 10, including an endoluminal delivery catheter 12 which, as shown in further detail below, has a lumen extending throughout its length, a distal end 14 and a proximal end 16. The catheter 12 and its lumen are typically, but not necessarily, circular in transverse cross-section. Disposed within the lumen of the catheter 12 is a first conductive electrical terminal 18, and in the preferred embodiment is able to slide within the catheter 12 so as to adjust the exposed operative length of the first terminal 18, as described in further detail below. At or proximate the distal 14 of the catheter 12 there is provided a second conductive terminal 20, which is shown disposed around the outer surface of the catheter 12.

The apparatus 10 also includes a proximal handle unit 22 coupled to the catheter 12 and the elongate terminal 18, as well as a control unit 24, the latter including first and second outputs 26, 28 coupled to feed wires 30, 32, which connect to respective ones of the electrical terminals 18, 20. The handle unit 22 can be gripped by a clinician during the deployment procedure, whereas the control unit 24 is used to control the application of current through the electrodes 18, 20 in order to effect ablation in the vessel. As described below, the control unit 24 may also include a motor for moving the elongate element 18 into and out of the catheter 12.

The apparatus 10 can be used to generate an electrical current within a vessel, depicted by current density lines 40 in Figure 1. The general method of operation of a bipolar RF ablation device is documented in the art and therefore in what follows is the description of the advances made to those general methods. In practice, when the electrodes 18, 20 are disposed within a vessel and energised appropriately, blood can be heated in the vessel to a temperature causing it to coagulate. On the other hand, when at least one of the electrodes, typically the electrode 18, is placed in contact with or within the tissue of the vessel wall, this will cause ablation and resultant shrinkage of the vessel wall tissue and thereby occlusion or closure of the vessel.

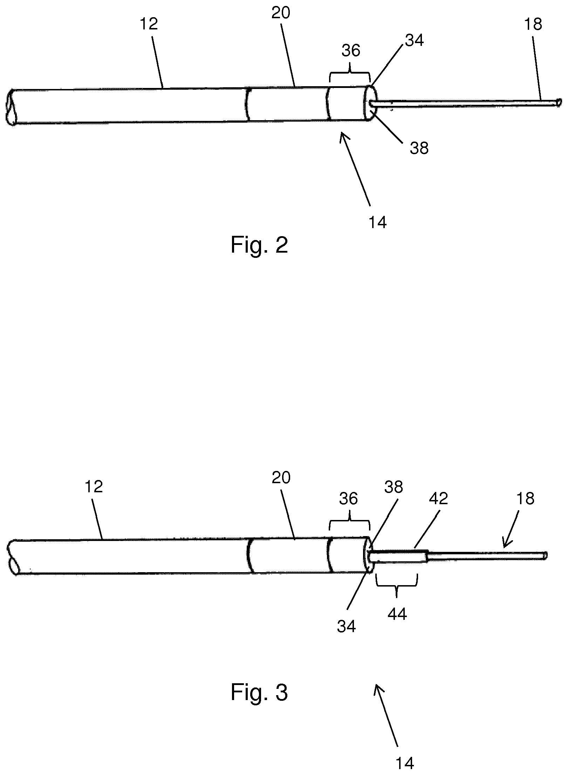

Referring now to Figure 2, this shows in better detail the distal end 14 of the ablation apparatus 10 of one embodiment of the invention. The second electrical terminal 20 is in the form of a sleeve which is disposed at the outer surface of the catheter 12 and spaced from the distal tip 34 of the catheter 12 by a gap 36. The gap 36 is preferably at least 2 mm but may be more, for example 3 mm. The gap 36 could have a length of up to 1 to 2 cm in some embodiments. The first conductive terminal 18 is in the form of a wire which fits within a lumen 38 of the catheter 12, as described above.

The two electrodes may have lengths in the region of 5 to 20 mm each, and more preferably in the region of around 10 mm. It is preferred though that the first electrode 18 is shorter than the second electrode 20. For these examples, the gap 36 may be in the region of 1-5 mm to provide, as described below, electrical isolation between the two electrodes 18, 20.

The electrode 20 has a significantly larger surface area than the electrode 18, preferably at least twice that of the electrode 18 and more preferably at least three times the surface area of the electrode 18. Having an electrode 20 with a surface area substantially larger than that of electrode 18 ensures that the electrode 20 does not heat appreciably during operation of the apparatus. Tests have established that when the surface area of the electrode 20 is two times that of the first electrode 18, the electrode 20 exhibits insignificant heating; while when it has a surface area three times (or more) the surface area of the first electrode 18, the second electrode 20 exhibits virtually no heating during operation of the device. As a consequence, heat energy is concentrated around the first electrode 18 and there is no or insignificant ablation occurring at the second electrical terminal 20, thereby leaving the catheter 12 free from ablated material, for example clotted blood.

With reference to Figure 2 in conjunction with Figure 1, the second electrical terminal 20 is coupled to feed wire 32 by a wire typically disposed within the body of the catheter 12, for instance within the wall forming the catheter 12. In some embodiments, where the catheter 12 has a strengthening element therewithin such as a strengthening coil or braid, the strengthening element could be used as the electrical conductor between the control system 24 and the electrical terminal 20. Any suitable insulated connecting wire may be used.

The electrical terminal 20, as the first electrical terminal 18, could be formed from any suitable conductive biocompatible material including, for example, platinum, gold, silver. The catheter 12 is preferably made of an insulating material, for instance polyurethane, nylon, PTFE or other suitable polymer material
With respect to Figure 2, the first electrical terminal 18 is uncoated, that is electrically exposed, at least along the length of the distal end thereof which in use extends beyond the distal tip 34 of the catheter 12. In practice, the first electrical terminal 18 may be entirely uncoated.

The second electrical terminal 20 may be bonded to the outer surface of the catheter 12, although is preferably within an indentation in the catheter 12, such that the outer surface of the electrical terminal 20 is flush with the outer surface of the catheter, so as not to present any sharp edges. The electrical terminal 20 may be a tight or friction fit on the catheter 12 or otherwise bonded thereto.

In the arrangement shown in Figure 2, therefore, the two electrical terminals 18, 20 are electrically isolated from one another as a result of the gap 36 separating the two.

Referring now to Figure 3, there is shown a slightly modified embodiment of assembly 10, which may differ solely by the provision of an insulating sleeve 42 over the first electrode 18 and which extends beyond the distal face 34 of the catheter 12 by a length 44, which may be anything from 1-5 mm for a structure having the dimensions described above. The sleeve 42 provides an insulating spacer between the electrodes 18, 20, in which case the second electrode 20 can be positioned to extend right to the distal face 34, that is to omit the gap, or spacer portion, 36 of the catheter 12.

The sleeve 42 may be fixed to the electrode 18 so as to be slidable into and out of the catheter 12 with the first electrode 18. In this configuration, the exposed length of the first electrode 18, and as a result its surface area, remains constant irrespective of how far the first electrode 18 is extended beyond the distal tip 34 of the catheter 12. This exposed length, or surface area, will only decrease once the entirety of the sleeve 42 has been withdrawn into the catheter 12 and the first electrode is then further withdrawn into the catheter 12.

In other embodiments, the sleeve 42 is fixed to the catheter 12 and the electrode 18 is slidable within the sleeve 42, such that there is always a constant length 44 of sleeve 42 at the distal end of the catheter 12, with the exposed length of the electrode varying in dependence upon the amount by which it is extended beyond the sleeve 42.

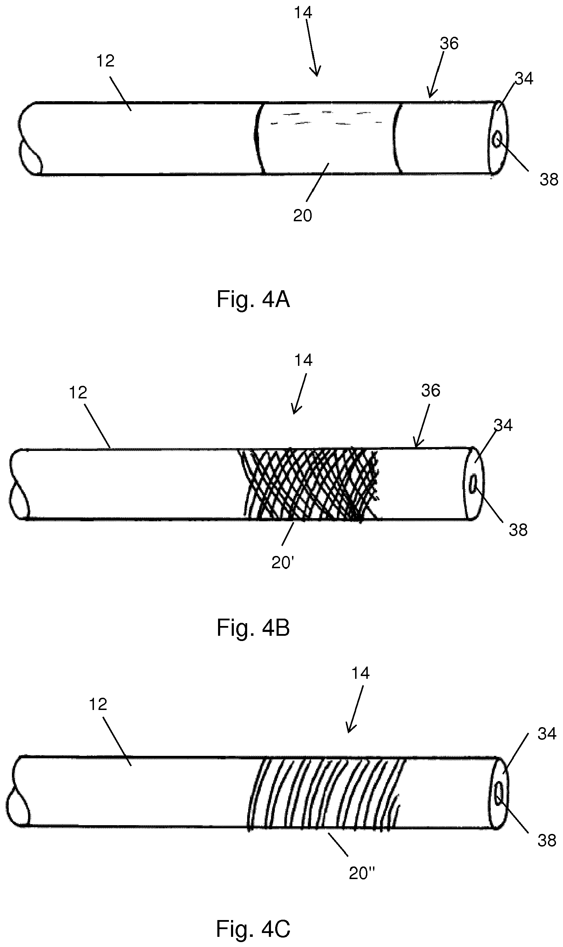

Referring now to Figures 4A to 4C, these show in enlarged form the distal end 14 of different embodiments of catheter assembly for the device of Figure 1. In Figure 4A, the second electrode 20 is in the form of a sleeve of conductive material, as described in the embodiments above. In Figure 4B, the second electrode 20' is a length of braiding on the outer surface of the catheter 12, the braiding being made of a conductive material. In Figure 4C, the second electrode 20" is a strip of conductive material wound helically around the outer surface of the catheter 12. The skilled person will appreciate that these are only examples of structure which can be used for the second electrode 20 and that other structures are equally suitable. A common factor in all of the examples is the provision of a second electrode 20 which has a surface area substantially greater than that of the first electrode 18 and preferably at least 2 to 3 times that of the first electrode 18. The conductive material may, for instance, be metal or a metal alloy.

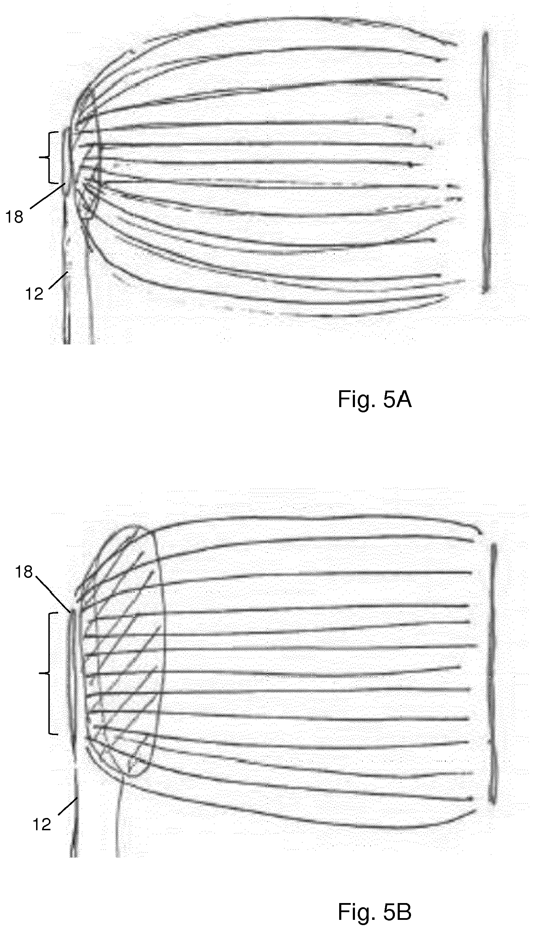

Referring now to Figures 5A and 5B, these depict the effect which different exposed lengths of the first electrode 18 have in use. In Figure 5A, there is a relatively short length of first electrode 18 exposed beyond the distal end of the catheter 12. This provides a high current density at the exposed part of the electrode 18, with a narrow field of heating; which can have in particular the following characteristics: 1) a low maximum current capacity before the focal field reaches a high temperature overheating the device, 2) focused local heating near the electrode, and 3) low total energy and as a result less collateral tissue damage.

Figure 5B, by contrast, shows a longer exposed length of first electrode 18, with the result that there is more even current density at the tip of the apparatus 10, resulting in a wider field of heating. This configuration provides the following characteristics: 1) a high maximum current capacity, 2) a wide field of heating, 3) risk of heating the second electrode 20, and 4) a high energy with deeper collateral tissue damage.

In different clinical situations there may be the need for different depths of heating. In the extreme case of tumour ablation, for example, it is desired to have a deep heating zone that extends into the tumour tissue, whereas in vessel embolization the heating zone should preferably be matched with the vessel size to minimize the amount of collateral damage to adjacent vessels, nerves or muscle tissue.

In RF heating the electrodes 18 and 20 form a resistive circuit with the blood and tissue. The feed wires and metal electrodes 18, 20 have negligible resistance, so essentially it is the current path through the tissue or blood that determines the circuit. Heat is generated due to Joule losses, that is current passing through a resistive medium, and is governed by the equation P=I^2^{∗}R, where P is the delivered power, I is the current and R is the resistance. Current is the dominant factor. In a three-dimensional field the concept may be expanded to current density and resistivity. By changing the electrode configuration, as depicted in Figures 5A and 5B, it is possible to change the current density distribution and hence the zone of heating. With larger electrode areas, the current density becomes homogenous and the field of heating more disperse. As the electrode becomes small, the current density becomes focused at the surface of the electrode and the electrode behaves more like a heating element.

A monopolar RF system is characterized by having one 'dispersive' electrode significantly larger than the other so that the current density at the disperse electrode is insignificant compared to that of the 'active' electrode. In this case, the field of heating at the active electrode is modulated by the area of that electrode alone, and not by the ratio of areas, as long as the dispersive electrode is significantly larger than the active electrode. In the applicant's experience, an electrode begins to behave as a dispersive electrode if it has an area of at least two to three times the active electrode, often at least three times. The effective area of an electrode may be compromised if is placed in the presence of poorly conductive tissue such as bone.

In a bipolar RF system the current passes between two electrodes. By keeping one electrode area constant and varying the area of the other electrode (as described herein), it is possible to change the field of heating from being focused at one electrode or the other or to be even across the two.

In both monopolar and bipolar applications the electrode configuration dictates a current density distribution and hence a relative distribution of the delivered power. The total amount of power can be adjusted by the RF generator. In a simple embodiment, for a given electrode area the appropriate maximum power setting may be empirically determined and provided in the instruction manual of the device.

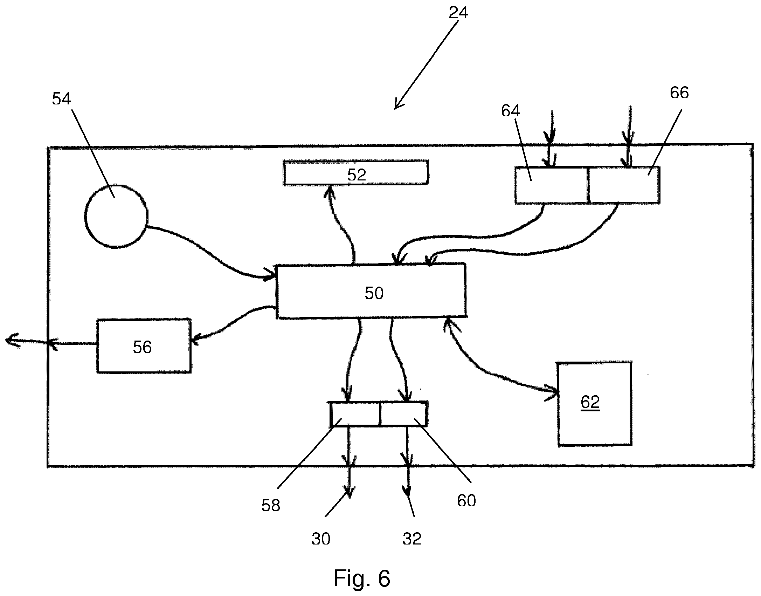

Referring now to Figure 6, there is shown in schematic form an embodiment of control unit 24 for the apparatus 10 of Figure 1. The person skilled in the art will appreciate that this is a schematic diagram showing only the principal components of such a control unit. The skilled person will also appreciate that the unit could have other structures and may also have other functionalities.

The example of control unit 24 shown in Figure 6 includes a processor 50 coupled to a display 52 for displaying the operating and/or control parameters of the assembly 10. The unit 24 may also include an input 54, which in this example can simply be a dial, for entering a measurement equivalent to the diameter of the vessel to be treated. A motor 56 is coupled to the first electrode 18, while outputs 58, 60 are coupled, respectively, to the feed wires 30, 32 for delivering current to the electrodes 18, 20. There is also provided a memory, such as a look up table 62 which includes data relating to the desired exposed length of the electrode 18 based on the treatment to be carried out and the inputted vessel size, in accordance with the teachings herein. The control unit 24 may also include inputs 64 and 66 for receiving probe inputs, such as from a temperature sensor or the like.

The control unit 24 is operable to adjust the total amount of power delivered through the electrodes 18, 20 into the vessel or material to be treated. For a given electrode area, the appropriate maximum power setting may be empirically determined and then stored in the look up table 62 for use by the processor 50. The skilled person will appreciate that in addition to having a data table providing values relating to the desired exposed surface area of the electrode 18 in connection with a variety of vessel sizes, the look up table 62 may also provide data relating to the amount of current to fed through the circuit, the length of time of application of current through the circuit, desired maximum temperature to be reached during the ablation process and/or the overall time for ablation. These values can all be determined empirically.

In a simple embodiment of the apparatus, a monopolar system may be used with a conducting electrode 18 inserted through an isolating catheter 12, with the exposed electrode area being manually adjustable by extending the electrode 18 beyond the distal face 34 of the catheter 12. In this embodiment, the device may be purely mechanical with no separate control system. It may, for instance, include distance markings on the electrode assembly 18 indicative of the exposed length of the electrode. The dispersive electrode may be a conventional grounding pad or an electrode situated on the distal portion of the catheter with an isolating region at the tip.

Workable dimensions for a system for very small arteries are 5 to 20mm² for the active electrode and 40mm² area for the dispersive electrode with an isolation region of around 2 mm (in the case of a catheter mounted dispersive electrode). The catheter could have an outer diameter of 1mm and the electrode an outer diameter of 0.5mm. Larger systems, such as 2.5mm diameter catheter are also envisioned.

The system may have a temperature sensor provided within the catheter adjacent its distal end.

All optional and preferred features and modifications of the described embodiments and dependent claims and clauses below are usable in all aspects of the invention taught herein. Furthermore, the individual features of the dependent claims and clauses below, as well as all optional and preferred features and modifications of the described embodiments are combinable and interchangeable with one another.

The disclosures in British patent application number 1505284.8, from which this application claims priority, in European patent application number 16275048.3 from which this application is divided, and in the abstract accompanying this application, are incorporated herein by reference.

According to aspects of the invention, there can be provided a medical ablation apparatus as in any of the following clauses.
1. Medical ablation apparatus including:
   an endoluminal delivery catheter having a lumen therein, an outer surface and a distal end wall, at least the outer surface of the catheter being made of an electrically non-conductive material;
   at least one elongate electrically conductive first terminal disposed in the lumen of the catheter, the first terminal having a first surface area;
   an electrically conductive second terminal disposed around the outer surface of the catheter and spaced from the first terminal so as to provide an insulating gap between the first and second terminals, the second terminal having a surface area a multiple of the surface area of the first terminal.
2. Medical ablation apparatus according to clause 1, wherein the second terminal is an electrically dispersive electrode and the electrically conductive first terminal is slidably disposed in the lumen of the catheter whereby to vary the operative length of the first terminal.
3. Medical ablation apparatus according to clause 2, wherein, at maximum exposure of the first terminal, the second terminal has a surface area a multiple of the surface area of the first terminal.
4. Medical ablation apparatus according to any preceding clause, wherein in operation the first terminal has an exposed length of at least 5mm.
5. Medical ablation apparatus according to any preceding clause, wherein the second terminal has a surface area at least three times the surface area of the first electrode.
6. Medical ablation apparatus according to any preceding clause, including a control system coupled to the first and second electrically conductive terminals, the control system including a motor unit connected to the first terminal and operable to move the first terminal relative to the second terminal, thereby to change an exposed length of the first terminal.
7. Medical ablation apparatus according to clause 6, wherein the control system includes a control input for inputting a vessel size, the control unit being configured to control the motor unit to adjust the exposed length of first terminal in dependence upon vessel size.
8. Medical ablation apparatus according to clause 7, wherein the control system includes a look-up table of vessel size and associated desired exposed length of the first electrode.
9. Medical ablation apparatus according to any preceding clause, wherein the first electrode is an electrically conductive wire.
10. Medical ablation apparatus according to any preceding clause, wherein the first electrode has length from 5 to 20 millimetres.
11. Medical ablation apparatus according to any preceding clause, wherein the first electrode has an exposed length from 8 to 15 millimetres.
12. Medical ablation apparatus according to any preceding clause, wherein the second electrode is an electrically conductive ring of braided conductive wires disposed around the catheter.
13. Medical ablation apparatus according to any one of clauses 1 to 11, wherein the second electrode is an electrically conductive winding of conductive strip material disposed helically around the catheter.
14. Medical ablation apparatus according to any preceding clause, wherein the first and second terminals are spaced from one another by an insulating gap of at least 3 millimetres.
15. Medical ablation apparatus according to any preceding clause, wherein the first and second terminals are spaced from one another by a sleeve disposed over a part of the first terminal.

## Claims

1. A method of configuring a medical ablation apparatus (10), the medical ablation apparatus including:
an endoluminal delivery catheter (12) having a lumen therein, an outer surface and a distal end wall (38), at least the outer surface of the catheter (12) being made of an electrically non-conductive material;
at least one elongate electrically conductive first terminal (18) disposed in the lumen of the catheter (12), the first terminal (18) having a first surface area;
an electrically conductive second terminal (20) disposed around the outer surface of the catheter (12) and spaced from an exposed operative length of the first terminal so as to provide an insulating gap (36) between the first (18) and second (20) terminals, the second terminal (20) having a surface area a multiple of the surface area of the first terminal (18); the method including:
configuring the first terminal to expose a desired operative length, wherein the desired operative length is selected on the basis of vessel size and/or flow speed;
wherein the method is not a method for treatment of the human or animal body by surgery.

2. A method according to claim 1, wherein the second terminal (20) is an electrically dispersive electrode and the electrically conductive first terminal (18) is slidably disposed in the lumen of the catheter (12) whereby to vary the operative length of the first terminal (18).

3. A method according to claim 2, wherein, at maximum exposure of the first terminal (18), the second terminal (20) has a surface area a multiple of the surface area of the first terminal.

4. A method according to any preceding claim, wherein the second terminal (20) has a surface area at least three times the surface area of the first electrode (18).

5. A method according to any preceding claim, including a control system (24) coupled to the first (18) and second (20) electrically conductive terminals, the control system (24) including a motor unit (56) connected to the first terminal (18) and operable to move the first terminal (18) relative to the second terminal (20), thereby to change an exposed length of the first terminal (18).

6. A method according to claim 5, wherein the control system (24) includes a control input (54) for inputting a vessel size, the control unit being configured to control the motor unit (56) to adjust the exposed length of first terminal (18) in dependence upon vessel size.

7. A method according to claim 6, wherein the control system (24) includes a look-up table of vessel size and associated desired exposed length of the first electrode (18).

8. A method according to any preceding claim, wherein the first electrode (18) is an electrically conductive wire.

9. A method according to any preceding claim, wherein the first electrode (18) has length from 5 to 20 millimetres.

10. A method according to any preceding claim, wherein the first electrode (18) has an exposed length from 8 to 15 millimetres.

11. A method according to any preceding claim, wherein the second electrode (20) is an electrically conductive ring of braided conductive wires disposed around the catheter (12).

12. A method according to any one of claims 1 to 10, wherein the second electrode (20) is an electrically conductive winding of conductive strip material disposed helically around the catheter (12).

13. A method according to any preceding claim, wherein the first (18) and second (20) terminals are spaced from one another by an insulating gap (36) of at least 3 millimetres.

14. A method according to any preceding claim, wherein the first (18) and second (20) terminals are spaced from one another by a sleeve (42) disposed over a part of the first terminal (18).
